# EUROPÄISCHE PATENTSCHRIFT

(11) **EP 3 120 178 B1**
(45) Veröffentlichungstag und Bekanntmachung des Hinweises auf die Patenterteilung: **23.08.2023**
(21) Anmeldenummer: 15710523.0
(22) Anmeldetag: 19.03.2015
(51) Int. Cl.: A61B 90/25, G02B 21/00, G02B 7/00

(54) **OPERATIONSMIKROSKOP-STATIV**
SURGICAL-MICROSCOPE STAND
PIED DE MICROSCOPE D'OPÉRATION

(30) Priorität: 19.03.2014 DE 102014103758
(43) Veröffentlichungstag der Anmeldung: 25.01.2017
(73) Patentinhaber: Leica Microsystems Schweiz AG, 9435 Heerbrugg (CH)
(72) Erfinder: SCHUTZ, Marco, CH-9443 Widnau (CH)
(74) Vertreter: Schaumburg und Partner Patentanwälte mbB
(86) Internationale Anmeldenummer: PCT/EP2015/055753
(87) Internationale Veröffentlichungsnummer: WO 2015/140241

(56) Entgegenhaltungen:
- EP-A1- 2 082 700
- EP-A2- 1 324 094
- DE-A1-102005 018 431
- DE-A1-102008 011 638
- JP-A- 2001 046 399
- US-A1- 2003 069 471

## Beschreibung

Die Erfindung betrifft ein Operationsmikroskop-Stativ nach dem Oberbegriff von Anspruch 1.

Bekannte Stative für Operationsmikroskope weisen eine Aufhängevorrichtung auf, welche insgesamt drei bewegliche Schlitten umfasst. Insbesondere sind ein sogenannter A-Schlitten, ein B-Schlitten und ein C-Schlitten vorgesehen, so dass eine Bewegung in alle drei Raumrichtungen möglich ist. Das Bewegen der Schlitten erfolgt hierbei über Motoren, wobei für das Bewegen jedes Schlittens ein eigener Motor vorgesehen ist. Beispielsweise beschreibt DE 10 2008 011 638 A1 ein Stativ für Operationsmikroskope, bei dem die einzelnen Schlitten motorisch verstellt werden können.

Neben dem automatischen Ausbalancieren können die einzelnen Schlitten auch manuell verfahren werden, wobei für jeden Schlitten an dem dazugehörigen Arm ein Kippschalter vorgesehen ist, mit dessen Hilfe der dazugehörige Schlitten in seine beiden Richtung verfahren werden kann um eine erneute Ausbalancierung bzw. eine Nachbalancierung zu verwirklichen.

Ein möglicher Ausbalancierungsmechanismus für ein Operationsmikroskop-Stativ ist dem Fachmann allgemein bekannt und wird hier nicht näher beschrieben.

Diese bekannten Stative haben den Nachteil, dass durch das Betätigen des Kippschalters beim manuellen Ausbalancieren jeweils ein Druck ausgeübt wird, wodurch ein genaues Ausbalancieren erschwert wird. Insbesondere, wenn das Operationsmikroskop-Stativ mit einem so genannten Drape umhüllt ist, wird ein erhöhter Druck bei Betätigung des Kippschalters benötigt, wodurch das manuelle Ausbalancieren nur äußerst ungenau erfolgen kann. Darüber hinaus haben die Kippschalter den Nachteil, dass sich in ihnen schnell Schmutz festsetzen kann und diese nur schwer hygienisch einwandfrei gereinigt werden können. Ein weiterer Nachteil dieser bekannten Stative ist es, dass je nach Position der Tragarme der Aufhängevorrichtung die Kippschalter nur schwer zugänglich sind und insbesondere die Kippschalter für unterschiedliche Schlitten sich an weit auseinander liegenden Stellen befinden. Dies führt zu einem bauraumintensiven und bewegungseinschränkenden Aufbau. Ein weiterer Nachteil dieser Stative ist es, dass die Motoren üblicherweise in jeder Lage der Tragarme beliebig betätigt werden können, was durch Überlastung der Motoren zu einem Überhitzen der Motoren führen kann. Hierdurch wird die Lebensdauer der Motoren eingeschränkt und es kann zu einer starken Geräuschemission kommen.

Es ist Aufgabe der Erfindung, ein Operationsmikroskop-Stativ anzugeben, das unter anderem eine einfache Bedienung und ein sicheres manuelles Ausbalancieren ermöglicht.

Diese Aufgabe wird durch ein Operationsmikroskop-Stativ mit den Merkmalen des Anspruchs 1 gelöst. Vorteilhafte Weiterbildungen der Erfindung sind in den abhängigen Ansprüchen angegeben.

Erfindungsgemäß umfasst das Operationsmikroskop-Stativ einen Bedienbereich, innerhalb welchem wenigstens eine Bedieneinheit zum manuellen Ansteuern der ersten und zweiten Antriebseinheiten vorgesehen ist. Durch Anordnen der wenigstens einen Bedieneinheit zum Steuern der ersten und zweiten Antriebseinheit innerhalb ein und desselben Bedienbereichs, wird ein kompakter Bedienbereich zum manuellen Ansteuern der Antriebseinheiten erzielt. Dies ermöglicht eine schnelle und effiziente (Nach)Balancierung während einer Operation. Des Weiteren bringt dies den Vorteil mit sich, dass der Nutzer des Statives sämtliche zur (Nach)Balancierung erforderlichen Bedienelemente durch Betrachten des einen Bedienbereichs im Blickfeld hat.

Üblicherweise wird vor Beginn einer Operation das Operationsmikroskop-Stativ automatisch ausbalanciert. Dies geschieht beispielsweise durch Bedienen eines Bedienelementes, welches in dem vorbeschriebenen Bedienbereich angeordnet sein kann. Es ist jedoch auch möglich, dieses Bedienelement zum automatischen Ausbalancieren des Stativs außerhalb des Bedienbereichs auszubilden. Besonders bevorzugt wird das Bedienelement für die automatische Ausbalancierung nicht nur an einem Bereich, sondern an mehreren Bereichen des Statives ausgebildet. Während einer Operation kann eine erneute Ausbalancierung notwendig sein, da durch beispielsweise Verstellen eines Mikroskoptubus sich der Schwerpunkt des Stativs verändert. Um diese Neubalancierung zu erzielen, steuert der Benutzer, in der Regel der Chirurg, den jeweiligen Schlitten manuell über ein vorgesehenes Bedienelement an. Für eine solche direkte und manuelle Ansteuerung des jeweiligen Schlittens ist an dem Stativ der vorgenannte Bedienbereich vorgesehen. Durch Anbringen der Bedienelemente für alle Schlitten innerhalb ein und demselben Bedienbereichs wird eine effiziente und kompakte Bauweise der Bedienelemente für die manuelle Ansteuerung erzielt und gleichzeitig eine vereinfachte und nutzerfreundliche Bedienung der jeweiligen Schlitten realisiert. Die Größe des Bedienbereichs kann beispielsweise der Fläche entsprechen, die erforderlich ist, um alle erforderlichen Bedienelement nebeneinander anzubringen.

Nach einer bevorzugten Ausführungsform der Erfindung umfasst das Stativ nur eine einzige Bedieneinheit, mit deren Hilfe sowohl die erste als auch die zweite Antriebseinheit manuell angesteuert werden können, also mit dessen Hilfe sowohl der erste als auch der zweite Schlitten bewegt werden können. Dies hat den Vorteil, dass nicht mehrere separate Bedieneinheiten für jeden Schlitten notwendig sind, sondern eine zentrale Bedienung aller Antriebseinheiten ermöglicht wird. Hierdurch wird der Bedienkomfort erhöht.

Nach einer weiteren Ausführungsform ist die Bedieneinheit zum manuellen Ansteuern der Schlitten durch ein in dem Bedienbereich angeordnetes Touchscreen ausgebildet. Die Verwendung eines Touchscreens hat den Vorteil, dass, verglichen mit Kippschaltern und ähnlichem, nur ein sehr geringer Druck ausgeübt werden muss und somit der manuelle Ausbalancierungsvorgang des Operationsmikroskop-Stativs nicht beeinträchtigt wird. Vorzugweise ist das Touchscreen ein resistiver Touchscreen. Solche Touchscreens können auch bei minimalem Druck durch ein Drape hindurch bedient werden. Ferner ermöglichen solche Touchscreens eine besonders gute Reinigung, so dass die Hygiene erhöht wird. Das Touchscreen bringt außerdem den Vorteil mit sich, dass nur relevante Informationen angezeigt werden können. Beispielsweise werden nur die Bedienfelder derjenigen Schlitten angezeigt, welche nach Berechnung einer Steuereinheit aufgrund ihrer aktuellen Lage auch bewegbar sind. Dies verhindert, dass ein Überfluss an Informationen an den Benutzer übermittelt wird. Dies hat den Vorteil, dass der Nutzer ein hohes Konzentrationsvermögen während der Operation aufrechterhalten kann. Die Größe des Bedienbereichs entspricht beim Realisieren der Bedienelemente über ein Touchscreen vorzugsweise der Größe (Fläche) des Touchscreens.

Gemäß einer besonders bevorzugten Ausführungsform der Erfindung umfasst das Operationsmikroskop-Stativ einen dritten Schlitten und eine dritte Antriebseinheit zum Antreiben des dritten Schlittens, wobei die Bedieneinheit zum manuellen Ansteuern des dritten Schlittens innerhalb des Bedienbereiches angeordnet ist. Somit ist auch die Bedieneinheit der dritten Antriebseinheit vorzugsweise innerhalb des Bedienbereichs angeordnet, wodurch ein Verfahren des Operationsmikroskopes in alle drei Richtungen durch Bedienen von lediglich einem einzigen Bedienbereich und vorzugsweise ein einziges Bedienelement ermöglicht wird.

An mindestens einem der Schlitten ist ein Lagesensor zur Ermittlung der Lage dieses Schlittens im Raum vorgesehen. Somit kann über den Lagesensor jederzeit genau die Position dieses Schlittens und hierüber auch die Position der anderen Schlitten bestimmt werden.

Der Lagesensor ist insbesondere an denjenigen Schlitten angeordnet, an dem das Mikroskop befestigt ist. Dies hat den Vorteil, dass mit Hilfe nur eines einzigen Lagesensors die Stellung aller Schlitten relativ zu einem Nullniveau bestimmt werden kann, da eine Lage jeweils eindeutig einer Stellung aller drei Schlitten zugeordnet werden kann.

Bei einer alternativen Ausführungsform der Erfindung können auch mehrere Lagesensoren vorgesehen sein. Insbesondere kann auch an jedem Schlitten jeweils ein eigener Lagesensor vorgesehen sein, über den jeweils nur die Lage des jeweiligen Schlittens ermittelt wird.

Es ist besonders vorteilhaft, wenn die mit Hilfe des Lagesensors ermittelte Lage über die Bedieneinheit einer Bedienperson angezeigt wird, so dass diese jederzeit die genaue Stellung der einzelnen Schlitten erkennen kann.

Für mindestens einen Schlitten ein vorbestimmter zulässiger Verstellbereich relativ zu einer Nullposition in einer Steuereinheit des Stativs gespeichert. Die Steuereinheit ist derart ausgelegt, dass sie nur ein Verfahren des Schlittens innerhalb dieses Verstellbereiches bei Bedienung der Bedieneinheit zulässt. Es ist besonders vorteilhaft, wenn für jeden Schlitten jeweils ein zulässiger Verstellbereich vorbestimmt ist und jeweils nur ein Verstellen innerhalb dieses Verstellbereiches möglich ist. Hierdurch wird erreicht, dass Überhitzungen vermieden werden und eine Betätigung der Antriebseinheit jeweils nur in dem Verstellbereich möglich ist und somit insbesondere keine Begrenzung der Bewegung der einzelnen Schlitten über Anschläge notwendig ist.

Die Steuereinheit vergleicht die mit Hilfe des Lagesensors ermittelte Lage mit dem vorbestimmten zulässigen Verstellbereich und schaltet in Abhängigkeit des Ergebnisses dieses Vergleichs ein Verstellen des jeweiligen Schlittens nur dann frei, wenn sich dieser innerhalb des zulässigen Verstellbereiches befindet. Insbesondere kann auch nur eine Verstellrichtung für den jeweiligen Schlitten in Abhängigkeit des Ergebnisses des Vergleiches freigeschaltet werden.

Das Freischalten kann insbesondere dadurch erfolgen, dass nur dann, wenn auch ein Verstellen zulässig ist, in dem Bedienbereich, vorzugsweise auf dem Touchscreen, ein entsprechendes Bediensymbol angezeigt wird, und in dem Fall, dass kein Verstellen zulässig ist, überhaupt keine Bediensymbole angezeigt werden. Alternativ können die Symbole auch immer angezeigt werden und in dem Fall, dass ein Verstellen nicht zulässig ist, anders gekennzeichnet sein. Beispielsweise können die Symbole grau hinterlegt werden oder blasser eingeblendet sein, so dass der Benutzer intuitiv weiß, dass diese Verstellung im Moment nicht zulässig ist.

Die Bedieneinheit zeigt Informationen darüber an, ob ein Verstellen des Schlittens in die jeweilige Richtung zulässig ist. Dies kann beispielsweise über einen Text oder über Symbole erfolgen.

Bei einer besonders bevorzugten Ausführungsform ermittelt eine Steuereinheit des Operationsmikroskop-Statives in Abhängigkeit von voreingestellten Kriterien und der jeweils in Echtzeit ermittelten Lage, welche Schlitten bewegt werden dürfen und welche nicht. Insbesondere ist die Steuereinheit so programmiert, dass Schlitten dann nicht verstellt werden dürfen, wenn sie so ausgerichtet sind, dass ein überhöhtes Gewicht auf die jeweilige Antriebseinheit lastet bzw. die Antriebseinheit eine überhöhte Antriebskraft erzeugen muss. Hierdurch wird eine Überbeanspruchung der jeweiligen Antriebseinheit vermieden. Insbesondere wird jeweils immer nur ein Schlitten angezeigt, der aktuell verstellt werden kann. Erst wenn dieser entsprechend verstellt ist bzw. die Arme entsprechend so gedreht sind, dass auf der Antriebseinheit des anderen Schlittens nicht mehr so ein großes Gewicht lastet, können auch die anderen Schlitten bzw. einer der anderen Schlitten für die Bedienung freigeschaltet werden.

Es ist besonders vorteilhaft, wenn die Bedieneinheit jeweils Informationen darüber anzeigt, welche Schlitten aktuell verstellt werden dürfen.

Bei dem Lagesensor handelt es sich insbesondere um einen Gravitationssensor, mit dessen Hilfe auf einfache Weise eine genaue Ermittlung der jeweiligen Lage möglich ist.

Die Verstellbereiche, innerhalb derer die Schlitten verstellt werden können, sind insbesondere über Lichtschranken abgesichert, d.h., dass mit Hilfe von Lichtschranken ermittelt wird, ob ein Schlitten am Ende eines Verstellbereiches angekommen ist und dann die entsprechende Antriebseinheit automatisch deaktiviert wird.

Ferner ist es vorteilhaft, wenn über die Bedieneinheit, insbesondere über den Touchscreen, weitere Informationen angezeigt werden. Insbesondere werden Informationen über die Ausbalancierung des Statives, die Beleuchtung, Videoaufnahmen, die Arbeitsdistanz, die Vergrößerung und/oder Warnhinweise angezeigt. Somit können mit Hilfe einer Bedieneinheit sämtliche Bewegung des Statives und gleichzeitig sämtliche Informationsausgaben in einem zentralen Ort erfolgen, wodurch die Bedienung wesentlich vereinfacht wird.

Ferner ist es vorteilhaft, wenn an einem der Schlitten eine Befestigungseinheit zur Befestigung eines Mikroskops vorgesehen ist und wenn der Bedienbereich oberhalb dieser Befestigungseinheit angeordnet ist. Hierdurch wird erreicht, dass die Bedieneinheit jederzeit einfach bedient und betrachtet werden kann, indem eine Bedienperson nur von dem eigentlichen Mikroskop nach oben schauen muss und Informationen erhält und alle Bedienungen durchführen kann.

Vorzugsweise ist der Bedienbereich an dem ersten Tragarm ausgebildet. Das hat unter anderem den Vorteil, dass ein Bewegen der Schlitten keinen Einfluss auf die Position des Bedienbereichs hat. Demnach bleibt bei der Betätigung des Bedienelements, vorzugweise des Touchscreens, innerhalb des Bedienbereichs der Bedienbereich trotzdem in seiner aktuellen Lage. Außerdem beeinflusst die Bedienung des Bedienbereichs den Ausbalancierungsvorgang selbst nicht, da der erste Tragarm einen neutralen Ort bildet. Der Druck, welcher auf Grund der Bedienung des Bedienbereichs durch den Benutzer auf das Stativ bzw. den Tragarm aufgebracht wird, übt kein Moment aus, welches bei der Neubalancierung berücksichtigt werden muss.

Weitere Merkmale und Vorteile der Erfindung ergeben sich aus der folgenden Beschreibung, die die Erfindung anhand von Ausführungsbeispielen in Zusammenhang mit den beigefügten Figuren näher erläutert.

Es zeigen:
- Figur 1: eine vereinfachte Darstellung eines bekannten Operationsmikroskop-Stativs;
- Figur 2: eine schematische, perspektivische Darstellung einer Aufhängevorrichtung eines Operationsmikroskop-Stativs gemäß einer ersten Ausführungsform der Erfindung; und
- Figur 3: eine schematische Darstellung einer Aufhängevorrichtung eines Operationsmikroskope-Stativs gemäß einer zweiten Ausführungsform der Erfindung.

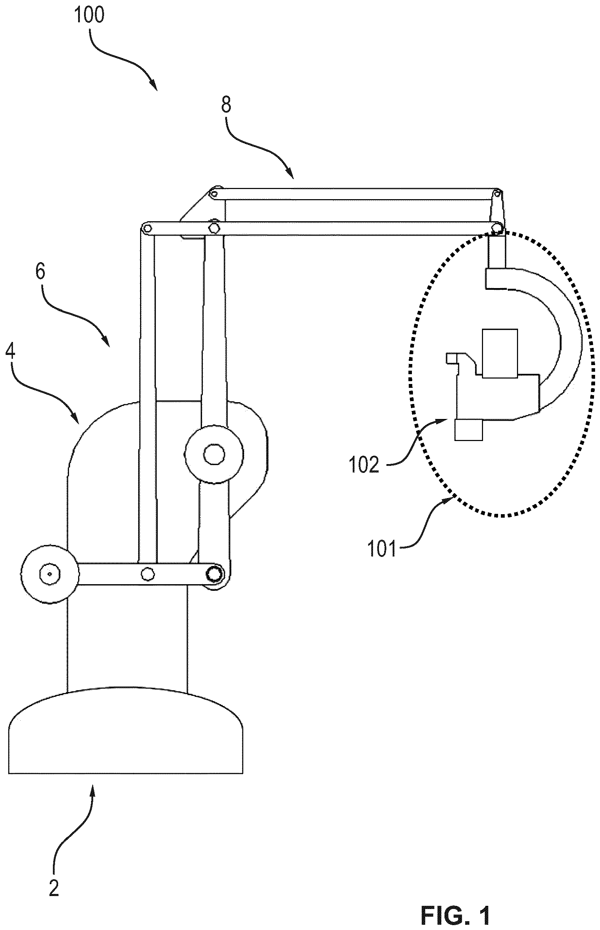

Figur 1 zeigt lediglich vereinfacht einen Teil des Aufbaus eines bekannten Operationsmikroskop-Stativs 100 mit einem Stativfuß 2, einer Trägersäule 4, einem ersten Lenkarm 6 und einem zweiten Lenkarm 8, wobei die Lenkarme 6,8 jeweils durch so genannte parallel Lenker gebildet sind. Am freien bzw. distalen Ende des zweiten Lenkarmes 8 ist eine Aufhängevorrichtung 101 aufgehängt, welche u.a. ein Operationsmikroskop 102 umfasst. Eine Steuereinheit (nicht dargestellt) ist beispielsweise in der Trägersäule 4 untergebracht.

Ein derartiger Aufbau ist beispielsweise aus der DE 10 2012 202 303 A1 bekannt, welche lediglich eine Möglichkeit eines Ausbalancierungsmechanismus beschreibt.

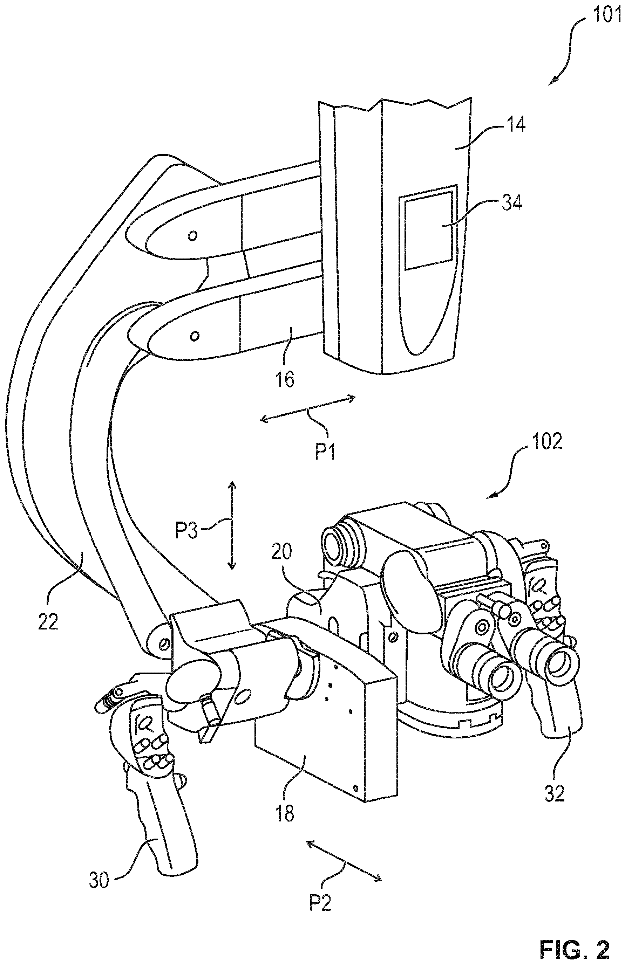

In Figur 2 ist eine schematische, perspektivische Darstellung einer Aufhängevorrichtung 101 eines Operationsmikroskope-Stativs gemäß einer ersten Ausführungsform der Erfindung dargestellt, welches ein Operationsmikroskop 102 umfasst.

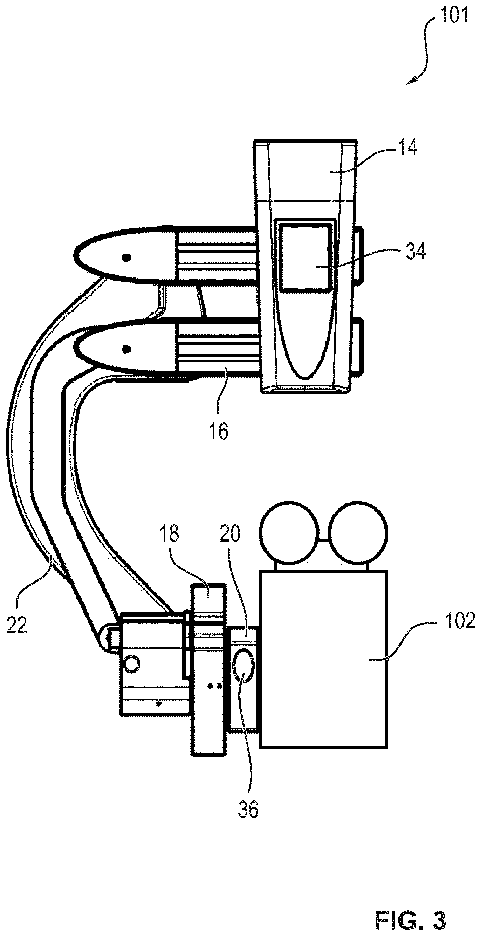

In Figur 3 ist eine schematische Darstellung einer Aufhängevorrichtung 101 gemäß einer zweiten Ausführungsform dargestellt, die der ersten Ausführungsform sehr ähnlich ist. Auch diese Aufhängevorrichtung 101 umfasst ein Operationsmikroskop 102. Elemente mit gleichem Aufbau oder gleicher Funktion haben dieselben Bezugszeichen.

Die Aufhängevorrichtung 101 umfasst einen ersten Tragarm 14 und einen zweiten Tragarm 22. Der erste Tragarm 14 ist mit seinem ersten Ende mit dem zweiten Lenkarm 8 und mit seinem anderem, zweiten Ende mit einem ersten Schlitten 16 verbunden.

Der erste Schlitten 16 ist relativ zum ersten Tragarm 14 in Richtung des Doppelpfeils P1 verfahrbar bzw. bewegbar. An dem ersten Schlitten 16 wiederum ist der zweite Tragarm 22 befestigt, der entsprechend zusammen mit dem ersten Schlitten 16 in Richtung des Doppelpfeils P1 bewegt wird. Der zweite Tragarm 22 ist insbesondere drehbar an dem ersten Schlitten 16 angeordnet.

An dem zweiten Tragarm 22 ist wiederum ein zweiter Schlitten 18 angeordnet, der in Richtung eines Doppelpfeils P2 relativ zu dem zweiten Tragarm 22 bewegbar gelagert ist. Ein weiterer, dritter Schlitten 20 ist direkt an dem zweiten Schlitten 18 befestigt und relativ zu diesem in Richtung des Doppelpfeils P3 verstellbar. Die Richtungen P1, P2 und P3 sind insbesondere alle orthogonal zueinander gerichtet, so dass ein Verstellen in alle Richtungen des Raumes möglich ist.

Über die Aufhängevorrichtung 101 mit den Schlitten 16, 18 und 20 wird eine Verstellbarkeit des Operationsmikroskopes 102 erreicht, so dass dieses an die jeweiligen Gegebenheiten bei der Operation und die Größe des Operateurs angepasst werden kann.

Das Verstellen der Schlitten 16, 18 und 20 erfolgt insbesondere über Antriebseinheiten, beispielsweise Elektromotoren, wobei jedem Schlitten 16, 18 und 20 ein eigener Motor zugeordnet ist, über den nur dieser verstellt wird. Die Motoren sind in den Figuren 1 bis 3 nicht sichtbar, da sie innerhalb des Gehäuses verdeckt angeordnet sind um Schädigungen vorzubeugen. Ferner umfasst das Stativ 100 gemäß der ersten Ausführungsform nach Figur 2 Griffe 30, 32, mit deren Hilfe das Operationsmikroskop 102 bewegt werden kann.

An dem ersten Tragarm 14 ist ein Bedienbereich ausgebildet, innerhalb welchem eine als Touchscreen 34 ausgebildete Bedieneinheit angeordnet ist. Über dieses Touchscreen 34 sind alle drei Motoren manuell ansteuerbar, so dass über die nur eine Bedieneinheit 34 das Bewegen aller Schlitten 16, 18 und 20 möglich ist. Bei diesem Ausführungsbeispiel entspricht die Größe des Bedienbereichs der Größe (bzw. Fläche) des Touchscreens 34.

Dies hat den Vorteil, dass die Anzahl der Bedieneinheiten reduziert wird und somit auch die Verkabelung und der gesamte Aufbau vereinfacht wird. Darüber hinaus wird der Bedienkomfort deutlich erhöht, da nicht, wie zuvor, für die Verstellung eines jeden Schlittens ein an ihm oder in seiner Nähe angeordnetes separates Bedienelement betätigt werden muss.

Die Anordnung des Touchscreens 34 an dem ersten Tragarm 14 hat ferner den Vorteil, dass ein Druck auf den Touchscreen 34 das Ausbalancieren des Operationsmikroskops nicht beeinträchtigt. Darüber hinaus bleibt die Position des Touchscreens 34 ortsfest, so dass sich die Bedienperson nicht auf eine veränderte Lage beim Verstellen des Operationsmikroskopes 102 einstellen muss.

Ferner wird durch die Verwendung eines Touchscreens 34 verglichen zu üblichen Kippschaltern oder ähnlichen Knöpfen eine deutliche höhere Hygiene erreicht, da ein solcher Touchscreen 34 einfach und rückstandslos gereinigt werden kann und keine Ritzen aufweist, in denen sich Schmutz festsetzen kann. Darüber hinaus kann ein solcher Touchscreen 34 auch dann einfach und zuverlässig bedient werden, wenn das Operationsmikroskop-Stativ durch ein so genanntes Drape umhüllt ist.

An dem dritten Schlitten 20 ist ein Lagesensor 36 vorgesehen, mit dessen Hilfe die Lage dieses dritten Schlittens 30 im Raum, insbesondere relativ zu einer vorbestimmten Null-Ausrichtung, ermittelt werden kann. Über die ermittelte Lage des dritten Schlittens 20 können auch die Lagen und Positionen der anderen Schlitten 16, 18 sowie der Tragarme der Aufhängevorrichtung bestimmt werden.

Die über den Lagesensor 36 ermittelte Lage wird insbesondere an die Steuereinheit übermittelt, welche in Abhängigkeit der ermittelten Lage des dritten Schlittens 20 die entsprechenden Ausrichtungen der anderen Schlitten 16, 18 bestimmt und die jeweilige Ausrichtung der einzelnen Schlitten 16 bis 20 mit voreingestellten Verstellbereichen vergleicht. Diese Verstellbereiche sind hierbei derart vorbestimmt, dass sie angeben, in welche Lage die einzelnen Schlitten 16 bis 20 manuell verstellt werden dürfen und bei welcher Lage sie entsprechend nicht bewegt werden sollen. Hierbei sind die Verstellbereiche insbesondere derart gewählt, dass nur dann die einzelnen Schlitten 16 bis 20 verstellt werden können, wenn auf ihnen ein möglichst geringes Gewicht lastet bzw. umgekehrt diejenigen Schlitten 16 bis 20 nicht verfahren werden dürfen, auf denen aufgrund ihrer aktuellen Ausrichtung ein überhöhtes Gewicht auf die des jeweiligen Schlittens 16 bis 20 zugeordnete Antriebseinheit lastet bzw. die Antriebseinheit eine erhöhte Antriebskraft erzeugen muss, um den Schlitten 16 bis 20 anzutreiben.

Darüber hinaus haben die Schlitten 16 bis 20 aufgrund ihrer Lagerung an bzw. den Tragarmen 14, 22 einen mechanisch vorgegebenen Stellbereich, innerhalb dessen sie verstellt werden können. Auch diese mechanisch begrenzte Verstellmöglichkeit ist innerhalb der in der Steuereinheit gespeicherten Verstellbereichen berücksichtigt, so dass die Steuereinheit nur dann ein Betätigen eines der Schlitten 16 bis 20 in eine der jeweiligen Richtungen P1 bis P3 erlaubt, wenn diese sich nicht bereits am Anschlag befindet. Dies kann insbesondere auch mit Hilfe von Lichtschranken überwacht und gesteuert werden.

Die Steuereinheit ist insbesondere derart programmiert, dass jeweils angezeigt wird, welcher Schlitten 16 bis 20 bzw. welche Schlitten 16 bis 20 aktuell verstellt werden dürfen. Die anderen Schlitten 16 bis 20, die nicht verstellt werden dürfen, werden insbesondere gar nicht angezeigt. Darüber hinaus werden über den Touchscreen 34 für denjenigen Schlitten, der verstellt werden darf, entsprechende Bedienelemente eingeblendet, bei deren Betätigung dieser Schlitten 16 bis 20 in seine entsprechende Richtung P1 bis P3 verfahren werden kann. Kann der Schlitten 16 bis 20 aufgrund seiner Stellung jedoch nur noch in einer Richtung verfahren, ist das andere Symbol insbesondere nur schwach eingeblendet oder grau hinterlegt, so dass die Bedienperson weiß, dass nur ein Verfahren in die eine Richtung möglich ist. Sofern gar kein Schlitten aufgrund der jeweiligen Positionen der Schlitten verstellt werden darf, wird dies dem Benutzer ebenfalls angezeigt.

Durch diese Beschränkung der Verstellmöglichkeiten der einzelnen Schlitten 16 bis 20 auf die jeweils aktuell sinnvollen Richtungen wird das Überhitzen der Motoren vermieden und somit deren Lebensdauer erhöht. Darüber hinaus wird eine unnötig hohe Geräuschentwicklung von vorneherein unterbunden.

Neben den einzelnen Verstellmöglichkeiten kann über das Touchscreen 34 auch eine Vielzahl weiter Informationen an die Bedienperson angezeigt werden. Beispielsweise kann angezeigt werden, ob sich das Operationsmikroskop-Stativ 100 in einem ausbalancierten Zustand befindet, wie die einzelnen Positionen der Schlitten 16 bis 20 aktuell sind, ob Beleuchtungen und/oder Videoaufnahmen aktiviert sind und/oder welche Arbeitsdistanz das Operationsmikroskop 102 aktuell aufweist. Darüber hinaus können über das Touchscreen 34 beispielsweise auch Warnhinweise angezeigt werden.

Somit erhält die Bedienperson über nur einen Touchscreen 34 zum einen die Bedienmöglichkeit aller Antriebseinheiten des Statives 100 und zum anderen einen zentralen Ort, über den ihr möglichst alle Informationen, die für sie relevant sind, angezeigt werden.

Eine übliche Benutzung des Operationsmikroskop-Stativs 100 wird nachfolgend kurz geschildert. Vor Beginn der Operation wird nach Aufbringen des erwünschten Operationsmikroskops 102 das Stativ 100 automatisch ausbalanciert. Dies geschieht mittels eines Knopfes der beispielsweise an der Trägersäule angeordnet sein kann. Ein solches Bedienelement kann jedoch auch über das Touchscreen 34 realisiert werden.

Nachdem die automatische Ausbalancierung durchgeführt worden ist, ist das Operationsmikroskop-Stativ einsatzbereit. Es kann nun während der Operation erwünscht sein eine Nachbalancierung durchzuführen, weil z.B. der Mikroskoptubus während der Operation verstellt wurde. Um eine erneute Ausbalancierung herzustellen, können nun die Schlitten 16 bis 20 direkt über das Touchscreen 34 angesteuert werden. In dem Touchscreen 34 werden Symbole zur Ansteuerung der jeweiligen Schlitten 16 bis 20 angezeigt und der Benutzer kann durch manuelle Eingabe den erwünschten Schlitten 16 bis 20 gezielt Bewegen um ein erneutes Ausbalancieren zu erzielen.

Der Nutzer muss lediglich einen Bedienbereich observieren und erhält hierdurch alle erforderlichen Informationen, um das Operationsmikroskop-Stativ schnell durch manuelles Ansteuern der jeweiligen Schlitten erneut auszubalancieren.

### Bezugszeichenliste

- 2: Stativfuss
- 4: Trägersäule
- 6,8: Lenkarme
- 14,22: Tragarm
- 16, 18, 20: Schlitten
- 30, 32: Griff
- 34: Touchscreen
- 36: Lagesensor
- 100: Operationsmikroskop-Stativ
- 101: Aufhängevorrichtung
- 102: Operationsmikroskop
- P1, P2, P3: Richtung

## Patentansprüche

1. Operationsmikroskope-Stativ (100) umfassend:
- einen an einem ersten Tragarm (14) angeordneten ersten Schlitten (16), der von einer ersten Antriebseinheit antreibbar ist; einem zweiten Tragarm (22) der an dem ersten Schlitten (16) angeordnet ist; und
- einem an dem zweiten Tragarm (22) angeordneten zweiten Schlitten (18), der von einer zweiten Antriebseinheit antreibbar ist;
- einen Bedienbereich (34), innerhalb welchem wenigstens eine Bedieneinheit zum manuellen Ansteuern der ersten und zweiten Antriebseinheiten angeordnet ist,
- wobei an mindestens einem der Schlitten (16, 18, 20) ein Lagesensor (36) zur Ermittlung der Lage im Raum angeordnet ist **gekennzeichnet durch**
- eine Steuereinheit in der für mindestens einen Schlitten (16, 18, 20) ein vorbestimmter zulässiger Verstellbereich relativ zu einer Nullposition in einer Steuereinheit des Stativs (100) gespeichert ist, wobei durch Betätigung der Bedieneinheit (34) ein Verfahren dieses Schlittens (16, 18, 20) nur innerhalb dieses Verstellbereichs möglich ist,
- die Steuereinheit die mit Hilfe des Lagesensors (36) ermittelte Lage mit dem vorbestimmten zulässigen Verstellbereich vergleicht und in Abhängigkeit des Ergebnisses dieses Vergleichs ein Verstellen des entsprechenden Schlittens (16, 18, 20) freischaltet und
- die Bedieneinheit Informationen anzeigt, ob ein Verstellen der Schlitten (16 bis 20) in die jeweiligen Richtungen (P1, P2, P3) zulässig ist.

2. Operationsmikroskop-Stativ (100) nach Anspruch 1, ferner umfassend einen dritten Schlitten (20) und eine dritte Antriebseinheit zum Antreiben des dritten Schlittens (20), wobei die Bedieneinheit zum manuellen Ansteuern des dritten Schlittens (20) innerhalb des Bedienbereiches (34) angeordnet ist.

3. Operationsmikroskop-Stativ (100) nach Anspruch 1 oder 2, wobei die Bedieneinheit zum manuellen Ansteuern der Schlitten (16, 18, 20) durch einen in dem Bedienbereich angeordneten Touchscreen (34) ausgebildet ist.

4. Operationsmikroskope-Stativ (100) nach einem der vorhergehenden Ansprüche, wobei der Bedienbereich (34) an dem ersten Tragarm (14) ausgebildet ist.

5. Operationsmikroskope-Stativ (100) nach einem der vorhergehenden Ansprüche, wobei der Lagesensor (36) an demjenigen Schlitten (16, 18, 20) angeordnet ist, an dem das Operationsmikroskop (102) befestigbar ist.

6. Operationsmikroskope-Stativ (100) nach einem der vorhergehenden Ansprüche, wobei die ermittelte Lage mit Hilfe der Bedieneinheit anzeigbar ist.

7. Operationsmikroskope-Stativ (100) nach einem der vorhergehenden Ansprüche, wobei für jeden Schlitten (16, 18, 20) jeweils ein vorbestimmter zulässiger Verstellbereich relativ zu einer Nullposition in der Steuereinheit gespeichert ist, und dass durch Betätigung der Bedieneinheit ein Verfahren des jeweiligen Schlittens (16, 18, 20) nur innerhalb des jeweiligen Verstellbereichs zulässt.

8. Operationsmikroskope-Stativ (100) nach einem der vorhergehenden Ansprüche, wobei die Steuereinheit in Abhängigkeit von in Echtzeit ermittelten Lagen der Schlitten festlegt, welche Schlitten (16, 18 20) bewegbar sind und eine Informationsanzeige an der Bedieneinheit entsprechend steuert.

9. Operationsmikroskope-Stativ (100) nach einem der vorhergehenden Ansprüche, wobei innerhalb des Bedienbereichs (34) eine Anzeigeeinheit vorgesehen ist, welche Informationen über die Ausbalancierung des Stativs, die Beleuchtung, Videoaufnahmen, die Arbeitsdistanz, die Vergrößerung und/oder Warnhinweise anzeigt.

10. Stativ nach einem der vorhergehenden Ansprüche, **dadurch gekennzeichnet, dass** an einem der Schlitten (16, 18, 20) eine Befestigungseinheit zur Befestigung eines Operationsmikroskops (102) angeordnet ist, und dass der Bedienbereich (34) oberhalb dieser Befestigungseinheit vorgesehen ist.

## Claims

1. A surgical microscope stand (100), comprising:
- a first carriage (16) arranged on a first support arm (14) and drivable by a first drive unit; a second support arm (22) arranged on the first carriage (16); and
- a second carriage (18) arranged on the second support arm (22) and drivable by a second drive unit;
- an operating area (34) within which at least one operating unit is arranged for manual control of the first and second drive units,
- wherein a position sensor (36) for determining the position in space is arranged on at least one of the carriages (16, 18, 20),
**characterized by** a control unit, in which for at least one carriage (16, 18, 20) a predetermined permissible adjustment range relative to a zero position is stored in a control unit of the stand (100), wherein by actuation of the operating unit (34), it is possible to move this carriage (16, 18, 20) only within this adjustment range,
- the control unit compares the position determined with the aid of the position sensor (36) with the predetermined permissible adjustment range and, depending on the result of this comparison, enables adjustment of the corresponding carriage (16, 18, 20), and
- the operating unit displays information on whether it is permissible to move the carriages (16 to 20) in the respective directions (P1, P2, P3).

2. The surgical microscope stand (100) according to claim 1, further comprising a third carriage (20) and a third drive unit for driving the third carriage (20), wherein the operating unit is arranged within the operating area (34) for manually controlling the third carriage (20).

3. The surgical microscope stand (100) according to claim 1 or 2, wherein the operating unit is designed for manual control of the carriages (16, 18, 20) by means of a touch screen (34) arranged in the operating area.

4. The surgical microscope stand (100) according to one of the preceding claims, wherein the operating area (34) is formed on the first support arm (14).

5. The surgical microscope stand (100) according to one of the preceding claims, wherein the position sensor (36) is arranged on that carriage (16, 18, 20) to which the surgical microscope (102) is attachable.

6. The surgical microscope stand (100) according to one of the preceding claims, wherein the determined position can be displayed with the aid of the operating unit.

7. The surgical microscope stand (100) according to one of the preceding claims, wherein for each carriage (16, 18, 20) in each case a predetermined permissible adjustment range relative to a zero position is stored in the control unit and that by actuation of the operating unit a movement of the respective carriage (16, 18, 20) is permitted only within the respective adjustment range.

8. The surgical microscope stand (100) according to one of the preceding claims, wherein the control unit determines which carriages (16, 18, 20) are movable as a function of positions of the carriages determined in real time and controls an information display on the operating unit accordingly.

9. The surgical microscope stand (100) according to one of the preceding claims, wherein a display unit is provided within the operating area (34), which displays information about the balance of the stand, the illumination, video recordings, the working distance, the magnification and/or warnings.

10. A stand according to one of the preceding claims, **characterized in that** a fastening unit for fastening a surgical microscope (102) is arranged on one of the carriages (16, 18, 20), and **in that** the operating area (34) is provided above this fastening unit.

## Revendications

1. Pied (100) de microscope opératoire comprenant :
- un premier coulisseau (16) agencé sur un premier bras porteur (14), qui est apte à être entraîné par une première unité d'entraînement ;
- un deuxième bras porteur (22) qui est agencé sur le premier coulisseau (16) ; et
- un deuxième coulisseau (18) agencé sur le deuxième bras porteur (22), qui est apte à être entraîné par une deuxième unité d'entraînement ;
- une zone de commande (34), à l'intérieur de laquelle est agencée au moins une unité de commande pour la commande manuelle des première et deuxième unités d'entraînement,
- un capteur de position (36) étant agencé sur au moins l'un des coulisseaux (16, 18, 20) pour déterminer la position dans l'espace, **caractérisé par**
- une unité de commande dans laquelle est mémorisée pour au moins un coulisseau (16, 18, 20) une plage de déplacement admissible prédéterminée par rapport à une position zéro dans une unité de commande du pied (100), un déplacement de ce coulisseau (16, 18, 20) n'étant possible qu'à l'intérieur de cette plage de déplacement par actionnement de l'unité de commande (34),
- l'unité de commande compare la position déterminée à l'aide du capteur de position (36) avec la plage de déplacement admissible prédéterminée et, en fonction du résultat de cette comparaison, autorise un déplacement du coulisseau correspondant (16, 18, 20) et
- l'unité de commande affiche des informations indiquant si un déplacement des coulisseaux (16 à 20) dans les directions respectives (P1, P2, P3) est autorisé.

2. Pied (100) de microscope opératoire selon la revendication 1, comprenant en outre un troisième coulisseau (20) et une troisième unité d'entraînement pour entraîner le troisième coulisseau (20), l'unité de commande pour commander manuellement le troisième coulisseau (20) étant agencée à l'intérieur de la zone de commande (34).

3. Pied (100) de microscope opératoire selon la revendication 1 ou la revendication 2, dans lequel l'unité de commande est conçue pour commander manuellement les coulisseaux (16, 18, 20) au moyen d'un écran tactile (34) agencé dans la zone de commande.

4. Pied (100) de microscope opératoire selon l'une des revendications précédentes, dans lequel la zone de commande (34) est formée sur le premier bras porteur (14) .

5. Pied (100) de microscope opératoire selon l'une des revendications précédentes, dans lequel le capteur de position (36) est agencé sur le coulisseau (16, 18, 20), sur lequel le microscope opératoire (102) est apte à être fixé.

6. Pied (100) de microscope opératoire selon l'une des revendications précédentes, dans lequel la position déterminée est apte à être affichée à l'aide de l'unité de commande.

7. Pied (100) de microscope opératoire selon l'une des revendications précédentes, dans lequel, pour chaque coulisseau (16, 18, 20), une plage de réglage admissible prédéterminée par rapport à une position zéro est enregistrée dans l'unité de commande, et dans lequel l'actionnement de l'unité de commande n'autorise un déplacement du coulisseau respectif (16, 18, 20) que dans la plage de réglage respective.

8. Pied (100) de microscope opératoire selon l'une des revendications précédentes, dans lequel l'unité de commande détermine, en fonction des positions des coulisseaux déterminées en temps réel, quels coulisseaux (16, 18, 20) sont mobiles et commande en conséquence un affichage d'informations sur l'unité de commande.

9. Pied (100) de microscope opératoire selon l'une des revendications précédentes, dans lequel une unité d'affichage est prévue à l'intérieur de la zone de commande (34), laquelle affiche des informations sur l'équilibrage du pied, l'éclairage, les enregistrements vidéo, la distance de travail, le grossissement et/ou les avertissements.

10. Pied selon l'une des revendications précédentes, **caractérisé en ce qu'**une unité de fixation destinée à fixer un microscope opératoire (102) est agencée sur l'un des coulisseaux (16, 18, 20), et **en ce que** la zone de commande (34) est prévue au-dessus de cette unité de fixation.
